# EUROPÄISCHE PATENTSCHRIFT

(11) **EP 2 722 323 B1**
(45) Veröffentlichungstag und Bekanntmachung des Hinweises auf die Patenterteilung: **28.11.2018**
(21) Anmeldenummer: 14151297.0
(22) Anmeldetag: 24.03.2011
(51) Int. Cl.: C07C 29/19, C07C 29/44, C07C 31/135, C07C 33/20, A61K 8/34, A61Q 19/00

(54) **Verfahren zur Herstellung von 2-Methyl-4-phenyl-2-pentanol**
Process for the preparation of 2-methyl-4-phenyl-2-pentanol
Procédé de production de 2-méthyl-4-phényl-2-pentanol

(30) Priorität: 24.03.2010 EP 10157654
(43) Veröffentlichungstag der Anmeldung: 23.04.2014
(62) Teilanmeldung aus: 11711827.3
(73) Patentinhaber: BASF SE, 67056 Ludwigshafen am Rhein (DE)
(72) Erfinder: Ebel, Klaus, 68542 Heddesheim (DE); Rüdenauer, Stefan, 67549 Worms (DE); Pelzer, Ralf, 37699 Fürstenberg (DE); Bock, Martin, 30167 Hannover (DE)

(56) Entgegenhaltungen:
- NICOLAS, M. ET AL.: "Preparation of some tertiary alcohols by electrochemical reduction followed by a coupling reaction of an acetone-olefin mixture", COMPTE RENDUS DES SEANCES DE L'ACADEMIE DES SCIENCES, SERIE C: SCIENCES CHIMIQUES, Bd. 265, Nr. 19, 1967, Seiten 1044-1047, XP009177897, ISSN: 0567-6541
- KAMITANAKA, T. ET AL.: "Direct addition of supercritical alcohols, acetone or acetonitrile to the alkenes without catalysts", TETRAHEDRON LETTERS, Bd. 48, Nr. 48, 2007, Seiten 8460-8463, XP022322876, ISSN: 0040-4039, DOI: 10.1016/J.TETLET.2007.09.159
- NAKAGAWA, T. ET AL.: "Reactions of supercritical alcohols with unsaturated hydrocarbons", JOURNAL OF SUPERCRITICAL FLUIDS, Bd. 27, Nr. 3, 2003, Seiten 255-261, XP004458590, ISSN: 0896-8446, DOI: 10.1016/S0896-8446(02)00269-3
- CHRISTOL, H. ET AL.: "TRANSPOSITIONS ACIDOCATALYSEES (XIE MEMOIRE). REACTION DE RITTER SUR LES ALCOOLS BENZYLIQUES TERTIAIRES", BULLETIN DE LA SOCIETE CHIMIQUE DE FRANCE, 1961, Seiten 2319-2324, XP009056866, ISSN: 0037-8968
- MARTSCHKE, R. ET AL.: "Rate Constants for the Addition of 2-Hydroxy-2-propyl Radicals to Alkenes in Solution studied by laser flash photolysis", HELVETICA CHIMICA ACTA, Bd. 80, Nr. 5, 1997, Seiten 1363-1374, XP55117326, ISSN: 0018-019X, DOI: 10.1002/hlca.19970800505

## Beschreibung

Die vorliegende Erfindung betrifft ein Verfahren zur Herstellung von 2-Methyl-4-phenyl-2-pentanol.

4-Cyclohexyl-2-methyl-2-butanol, das auch als Coranol bezeichnet wird, ist ein Riechstoff mit einem Maiglöckchenduft, dessen Verwendung als Bestandteil von Duftzusammensetzungen erstmalig in US 4,701,278 beschrieben wurde.

Die Herstellung von 4-Cyclohexyl-2-methyl-2-butanol wurde von N.E. Okazawa et al. in Can. J. Chem. 60 (1982), 2180-93 beschrieben und umfasst die Umwandlung von 3-Cyclohexylpropansäure in ihr Säurechlorid, das anschließend mit 2 mol Methyllithium zum 4-Cyclohexyl-2-methyl-2-butanol umgesetzt wird. Aufgrund der Verwendung von Methyllithium ist dieses Herstellungsverfahren, insbesondere bei der Durchführung in größerem Maßstab, mit nicht unbeträchtlichen Risiken behaftet und wirtschaftlich unattraktiv. Dennoch wurden bislang in der Literatur keine weiteren Herstellungsverfahren beschrieben.

Nicolas et al., Compte Rendus des Seances de l'Academie des Sciences, Serie C: Sciences Chimiques, Bd. 265, Nr. 19 (1967), S. 1044-1047, beschreiben die Herstellung von 2-Methyl-4-phenyl-2-pentanol durch eine reduktive elektrochemische Kupplung von Aceton an α-Methylstyrol bei einer Elektrolyttemperatur von 30°C, wobei 2-Methyl-4-phenyl-2-pentanol in einer Ausbeute von 24% erhalten wird.

Christol et al., Bulletin de la Societe Chimique de France (1961), S. 2319-2324, beschreiben ein Verfahren zur Herstellung von 2-Methyl-4-phenyl-2-pentanol, bei dem zunächst Crotonsäure in Gegenwart von AlCl₃ an Benzol kondensiert wird, unter Erhalt von 3-Phenylbuttersäure. Die so erhaltene 3-Phenylbuttersäure wird anschließend in den Ethylester überführt und dann mit Methylmagnesiumbromid umgesetzt, wobei 2-Methyl-4-phenyl-2-pentanol in einer Ausbeute von 40% erhalten wird.

Die Erfindung betrifft ein Verfahren zur Herstellung von 2-Methyl-4-phenyl-2-pentanol, umfassend die Umsetzung von α-Methylstyrol mit Isopropanol bei einer Temperatur im Bereich von 250 bis 500 °C und einem Druck im Bereich von 5 bis 50 MPa, wobei man 2-Methyl-4-phenyl-2-pentanol erhält, wobei das Molverhältnis des eingesetzten α-Methylstyrols zu dem eingesetzten Isopropanol im Bereich von 1 : 5 bis 1 : 100 liegt. Bei dem erfindungsgemäßen Verfahren wird Isopropanol mit α-Methylstyrol bei erhöhter Temperatur umgesetzt. Hierbei bildet sich im Sinne einer Hydroxyalkylierung 2-Methyl-4-phenyl-2 -pentanol.

Über ähnliche Umsetzungen wurde von T. Nakagawa et al. im Rahmen einer analytischen Untersuchung zur Umsetzung von Styrol mit Alkanolen unter überkritischen Bedingungen berichtet (siehe J. Supercritical Fluids, 27 (2003), S. 255-261 und Tetrahedron Lett., 48 (2007), S. 8460-8463). Die Reaktion wurde jedoch bislang nicht zur präparativen Gewinnung von 2-Methyl-4-phenyl-2-pentanol genutzt.

Im Hinblick auf die Selektivität der Reaktion hat es sich als vorteilhaft erwiesen, wenn man das erfindungsgemäße Verfahren unter überkritischen Bedingungen durchführt. Hierunter versteht man Reaktionsbedingungen, bei denen wenigstens eine der Komponenten der Reaktionsmischung, vorzugsweise das Isopropanol, im überkritischen Zustand vorliegt. Dementsprechend erfolgt in dem erfindungsgemäßen Verfahren die Umsetzung unter Bedingungen, bei denen Isopropanol im überkritischen Zustand vorliegt. Die kritische Temperatur T_{c} von Isopropanol ist 235 °C, der kritische Druck P_{c} beträgt 4,8 MPa. Überkritische Bedingungen können vom Fachmann durch Variation von Druck und Temperatur eingestellt werden.

Die für eine hinreichende Geschwindigkeit der Umsetzung von α-Methylstyrol mit Isopropanol erforderliche Temperatur beträgt in der Regel wenigstens 250 °C, häufig wenigstens 300 °C und insbesondere wenigstens 320 °C. Zur Erzielung einer hinreichenden Selektivität der Reaktion hat es sich als vorteilhaft erwiesen, wenn die Temperatur der Umsetzung einen Wert von 500 °C, insbesondere 400 °C nicht überschreitet. Das erfindungsgemäße Verfahren erfolgt bei erhöhtem Druck, der in der Regel im Bereich von 5 bis 50 MPa, häufig im Bereich von 10 bis 30 MPa und insbesondere im Bereich von 15 bis 25 MPa. Der Druck im Reaktionsgefäß kann durch Beaufschlagung mit einer Inerten eingestellt werden. Vorzugsweise erfolgt die Umsetzung unter dem bei der gewünschten Reaktionstemperatur herrschenden Eigendruck der Reaktionsmischung.

Die Reaktionsdauer hängt naturgemäß von den gewählten Bedingungen und dem gewünschten Umsatz ab und liegt üblicherweise im Bereich von 30 sec. bis 4 h, insbesondere im Bereich von 3 min. bis 3 h und speziell im Bereich von 5 min bis 2,5 h. In der Regel wird die Reaktion soweit geführt, dass der im Unterschuss eingesetzte Reaktand, bei dem es sich um α-Methylstyrol handelt, zu wenigstens 80 %, insbesondere zu wenigstens 90 % umgesetzt ist.

In einer Ausführungsform der Erfindung liegt die Reaktionsdauer im Bereich von 30 min. bis 4 h, insbesondere im Bereich von 1 bis 3 h und speziell im Bereich von 1,5 bis 2,5 h. In der Regel wird die Reaktion soweit geführt, dass der im Unterschuss eingesetzte Reaktand, bei dem es sich um α-Methylstyrol handelt, zu wenigstens 80 %, insbesondere zu wenigstens 90 % umgesetzt ist.

Als besonders vorteilhaft hat es sich erwiesen, das erfindungsgemäße Verfahren bei erhöhten Temperaturen, d.h. oberhalb 300°C, insbesondere oberhalb 320°C, bevorzugt im Bereich 350 °C und 400 °C durchzuführen. Dies erlaubt kurze Reaktionszeiten, die üblicherweise im Bereich von 30 sec. bis 30 min, insbesondere im Bereich von 3 min bis 20 min und speziell im Bereich von 5 min bis 15 min liegen. Auf diese Weise können auch bei hohem Umsatz an α-Methylstyrol Selektivitäten bezüglich des Zielproduktes von deutlich > 60 % erzielt werden.

Im Hinblick auf die Selektivität der Reaktion hat es sich als vorteilhaft erwiesen, wenn man das erfindungsgemäße Verfahren in weitgehender oder vollständiger Abwesenheit von Katalysatoren, wie beispielsweise Radikalstarter, Säuren oder Übergangsmetallverbindungen durchführt. Weitgehende Abwesenheit bedeutet, dass die Konzentration etwaige Katalysatoren weniger als 1 g/kg (< 1000 ppm), insbesondere weniger 0,1 g/kg (< 100 ppm) bezogen auf das Gesamtgewicht der Reaktionsmischung, beträgt.

Die Umsetzung von α-Methylstyrol mit Isopropanol kann in Substanz oder in einem geeigneten, d. h. unter Reaktionsbedingungen inerten, Verdünnungsmittel durchgeführt werden. Geeignete inerte Verdünnungsmittel sind aprotische organische Lösungsmittel, die keine ethylenisch ungesättigte Doppelbindung aufweisen, wie beispielsweise aliphatische und alicyclische Ether mit vorzugsweise 4, 5 oder 6 C-Atomen, aliphatische und cycloaliphatische gesättigte Kohlenwasserstoffe mit vorzugsweise 6 bis 8 C-Atomen, Alkylester aliphatischer Carbonsäuren mit vorzugsweise 4 bis 8 C-Atomen und Gemische der vorgenannten Lösungsmittel. Vorzugsweise erfolgt das erfindungsgemäße Verfahren in Substanz, d. h. zur Umsetzung werden im Wesentlichen keine von α-Methylstyrol und Isopropanol verschiedenen Einsatzstoffe, wie beispielsweise inerte Lösungsmittel, eingesetzt. Im Wesentlichen bedeutet hier, dass α-Methylstyrol und Isopropanol wenigstens 95 Gew.-%, insbesondere wenigstens 99 Gew.-%, bezogen auf die Gesamtmenge der eingesetzten Komponenten, ausmachen. Daneben können die zur Reaktion eingesetzten Reaktanden, d. h. α-Methylstyrol und Isopropanol, herstellungsbedingt geringe Mengen an Verunreinigungen wie Wasser, Ethylbenzol, Toluol und dergleichen enthalten, wobei die Verunreinigungen in der Regel weniger als 5 Gew.-%, insbesondere weniger als 1 Gew.-%, bezogen auf die Gesamtmenge der Reaktanden ausmachen. Insbesondere beträgt der Wassergehalt der eingesetzten Reaktanden nicht mehr als 1 Gew.-%, bezogen auf die Gesamtmenge der Reaktanden.

Im Hinblick auf die Selektivität der Reaktion hat es sich als vorteilhaft erwiesen, wenn man in dem erfindungsgemäßen Verfahren Isopropanol in großem Überschuss, bezogen auf α-Methylstyrol einsetzt und/oder dafür Sorge trägt, dass in der Reaktionszone, in der α-Methylstyrol und Isopropanol unter Reaktionsbedingungen miteinander in Kontakt gebracht werden, ein hoher Überschuss an Isopropanol, bezogen auf das in der Reaktionszone befindliche α-Methylstyrol vorliegt. In der Regel setzt man in dem erfindungsgemäßen Verfahren α-Methylstyrol und Isopropanol in einem Molverhältnis α-Methylstyrol zu Isopropanol von höchstens 1 : 5, vorzugsweise höchstens 1 : 10, insbesondere höchstens 1 : 30, besonders bevorzug höchstens 1 : 40 und speziell höchstens 1 : 50 um. Im Hinblick auf eine effiziente Reaktionsführung setzt man in dem erfindungsgemäßen Verfahren α-Methylstyrol und Isopropanol in einem Molverhältnis im Bereich von 1 : 5 bis 1 : 100 und speziell im Bereich von 1 : 50 bis 1 : 90 ein.

Das erfindungsgemäße Verfahren kann absatzweise durchgeführt werden (so genannte batch-Fahrweise), d. h. α-Methylstyrol und Isopropanol werden im gewünschten Molverhältnis in einem geeigneten Reaktor vorgelegt und auf die gewünschten Reaktionsbedingungen gebracht und bis zu dem gewünschten Umsatz unter Reaktionsbedingungen gehalten. Das erfindungsgemäße Verfahren kann auch in der so genannten semi-batch-Fahrweise durchgeführt werden, d. h. die Hauptmenge, in der Regel wenigstens 80 %, einer oder beide Reaktanden, wird kontinuierlich oder portionsweise über einen längeren Zeitraum, in der Regel wenigstens 50 % der gesamten Umsatzdauer, unter Reaktionsbedingungen in den Reaktor gegeben. Das erfindungsgemäße Verfahren kann auch kontinuierlich durchgeführt werden, d. h. α-Methylstyrol und Isopropanol werden im gewünschten Molverhältnis kontinuierlich in eine Reaktionszone eingespeist und das Reaktionsgemisch wird kontinuierlich der Reaktionszone entnommen. Die Rate, mit der α-Methylstyrol und Isopropanol der Reaktionszone zugeführt werden, richtet sich nach der gewünschten Verweilzeit, die ihrerseits in bekannter Weise von der Reaktorgeometrie abhängt und der oben angegebenen Umsetzungsdauer entspricht.

Das erfindungsgemäße Verfahren kann grundsätzlich in allen Reaktoren durchgeführt werden, die für die gewählten Reaktionsbedingungen geeignet sind, vorzugsweise in Autoklaven, die Vorrichtungen zur Durchmischung der Reaktanden aufweisen können, oder in Reaktionsrohren.

Um das Molverhältnis von α-Methylstyrol zu Isopropanol während der Umsetzung niedrig zu halten und gleichzeitig eine effiziente Reaktionsführung zu erlauben, hat es sich als vorteilhaft erwiesen, wenn man wenigstens 80 %, insbesondere wenigstens 90 % des eingesetzten Isopropanols gegebenenfalls zusammen mit einer Teilmenge des α-Methylstyrols vorlegt und wenigstens 80 %, insbesondere wenigstens 90 % des eingesetzten α-Methylstyrols der Umsetzung unter Reaktionsbedingungen zuführt. Die Zugabe des α-Methylstyrols kann portionsweise oder vorzugsweise kontinuierlich erfolgen. Die Geschwindigkeit, mit der man α-Methylstyrol zuführt wird dabei vorzugweise so gewählt, dass das Molverhältnis des in die Reaktionszone bzw. den Reaktor eingespeisten, noch nicht abreagierten α-Methylstyrols zu dem in der Reaktionszone befindlichen Isopropanol während der Umsetzung weniger als 1 : 10 beträgt, insbesondere nicht mehr als 1 : 40 und speziell nicht mehr als 1 : 50 beträgt, z. B. im Bereich von < 1 : 10 bis 1 : 2000, vorzugsweise im Bereich von 1 : 40 bis 1 : 1500 und insbesondere im Bereich von 1 : 50 bis 1 : 1000 liegt. Bei einer kontinuierlichen Reaktionsführung wird man daher vorzugsweise α-Methylstyrol und Isopropanol in den zuvor genannten Molverhältnissen dem Reaktor bzw. der Reationszone zuführen. In einer bestimmten Ausführungsform der Erfindung wird die Geschwindigkeit, mit der man α-Methylstyrol zuführt vorzugweise so gewählt, dass das Molverhältnis des in die Reaktionszone bzw. den Reaktor eingespeisten, α-Methylstyrols zu dem in der Reaktionszone befindlichen Isopropanol im Bereich von 1:10 bis 1:130, insbesondere im Bereich von 1 : 20 bis 1 : 120, besonders bevorzugt im Bereich von 1:40 bis 1:100 und speziell im Bereich von 1 : 50 bis 1 : 90 liegt. Dies gilt insbesondere auch bei kontinuierlicher Reaktonsführung für die dem Reaktor bzw. der Reaktionszone zugeführten Molverhältnisse von α-Methylstyrol und Isopropanol.

Das im erfindungsgemäßen Verfahren erhaltene Reaktionsgemisch kann in an sich bekannter Weise aufgearbeitet werden oder unmittelbar als solches in eine heterogen-katalytische Hydrierung eingesetzt werden. In der Regel hat es sich als vorteilhaft erwiesen, das im erfindungsgemäßen Verfahren anfallende Reaktionsgemisch aufzuarbeiten, beispielsweise extraktive oder destillativ oder durch eine Kombination dieser Maßnahmen. In einer Ausführungsform des erfindungsgemäßen Verfahrens arbeitet man das im erfindungsgemäßen Verfahren anfallende Reaktionsgemisch destillativ auf, wobei man das gewünschte 2-Methyl-4-phenyl-2-pentanol als Mittelfraktion von Leicht- und Schwersiedern abtrennt. Sofern mit einem Isopropanol-Überschuss gearbeitet wird, kann das die Leichtsieder-Fraktion, die überwiegend aus Isopropanol besteht, in den Prozess zurückgeführt werden. In der Regel wird man vor einer heterogen-katalytische Hydrierung Isopropanol weitgehend entfernen, so dass der Anteil an Isopropanol in dem zur Hydrierung eingesetzten Edukt weniger als 20 Gew.-%, insbesondere nicht mehr als 10 Gew.-%, bezogen auf die Gesamtmenge an Edukt in der heterogen-katalytische Hydrierung, beträgt.

Das im erfindungsgemäßen Verfahren gewonnene 2-Methyl-4-phenyl-2-pentanol kann anschließend einer heterogen-katalytischen Hydrierung an einem zur Kernhydrierung von Aromaten geeigneten Katalysator, der im Folgenden auch als Kontakt bezeichnet wird, unterworfen werden.

Geeignete Kontakte sind grundsätzlich alle Kontakte, die bekanntermaßen zur Kernhydrierung von Aromaten geeignet sind, d. h. Kontakte, welche die Hydrierung von Phenylgruppen zu Cyclohexylgruppen katalysieren. Hierbei handelt es sich üblicherweise um Kontakte, die wenigstens ein Aktivmetall aus der Gruppe VIIIB des Periodensystems (CAS-Version) enthalten, wie z. B. Palladium, Platin, Cobalt, Nickel, Rhodium, Iridium, Ruthenium, insbesondere Ruthenium, Rhodium oder Nickel, oder ein Gemisch aus zwei oder mehr davon enthalten, gegebenenfalls in Kombination mit einem oder mehreren weiteren Aktiv-Metallen. Bevorzugte weitere Aktivmetalle sind aus den Gruppen IB oder VIIB des Periodensystems (CAS-Version) ausgewählt. Unter den ebenfalls verwendbaren Metallen der Nebengruppen IB und/oder VIIB des Periodensystems der Elemente sind z. B. Kupfer und/oder Rhenium geeignet.

Bei den Katalysatoren kann es sich um Vollkatalysatoren oder vorzugsweise um Trägerkatalysatoren handeln. Geeignete Trägermaterialien sind beispielsweise Aktivkohle, Siliciumcarbid, Siliciumdioxid, Aluminiumoxid, Magnesiumoxid, Titandioxid, Zirkondioxid, Alumosilicate und Gemische dieser Trägermaterialien. Die Menge an Aktivmetall beträgt üblicherweise 0,05 bis 10 Gew.-%, häufig 0,1 bis 7 Gew.-% und insbesondere 0,1 bis 5 Gew.-%, bezogen auf das Gesamtgewicht des Trägerkatalysators, insbesondere, wenn es sich bei dem Aktivmetall um ein Edelmetall wie Rhodium, Ruthenium, Platin, Palladium oder Iridium handelt. Bei Katalysatoren, die als Aktivmetalle Cobalt und/oder Nickel enthalten, kann die Menge an Aktivmetall bis zu 100 Gew.-% betragen und liegt üblicherweise im Bereich von 1 bis 100 Gew.-%, insbesondere 10 bis 90 Gew.-% bezogen auf das Gesamtgewicht des Katalysators.

Die Trägerkatalysatoren können in Form eines Pulvers eingesetzt werden. In der Regel weist ein solches Pulver Teilchengrößen im Bereich von 1 bis 200 µm insbesondere 1 bis 100 µm auf. Pulverförmige Katalysatoren eignen sich insbesondere dann, wenn der Katalysator in der zu hydrierenden Reaktionsmischung suspendiert wird (Suspensionsfahrweise). Bei Einsatz des Katalysatoren in Katalysatorfestbetten verwendet man üblicherweise Formkörper, die z. B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt. Ebenfalls bevorzugt sind Kugelförmige Trägermaterialien mit Kugeldurchmessern im Bereich von 1 bis 10 mm, insbesondere 2 bis 6 mm.

Geeignete Kontakte sind solche, die wenigstens ein unter Ruthenium, Rhodium und Nickel ausgewähltes Aktivmetall und gegebenenfalls in Kombination mit einem oder mehreren weiteren Aktivmetallen, die aus den Gruppen IB, VIIB oder VIIIB des Periodensystems (CAS-Version) ausgewählt sind.

Besonders geeignete Kontakte sind Ruthenium-haltige Kontakte. Diese enthalten Ruthenium als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle aus der Gruppe VIIIB sind z. B. Platin, Rhodium, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. Bevorzugt wird Ruthenium alleine als Aktivmetall oder zusammen mit Platin oder Iridium als Aktivmetall eingesetzt; ganz besonders bevorzugt wird Ruthenium alleine als Aktivmetall eingesetzt.

Geeignet sind insbesondere Ruthenium-haltige Kontakte, in den das Ruthenium auf einem Trägermaterial angeordnet ist, sog. Ruthenium-haltige Trägerkatalysatoren. Die Trägermaterialien derartiger Trägerkatalysatoren weisen in der Regel eine spezifischen BET-Oberfläche, bestimmt durch N₂-Adsorbtion nach DIN 66131,von wenigstens 30 m²/g, insbesondere 50 bis 1000 m²/g auf. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen.

Geeignete Ruthenium-haltige Kontakte sind beispielsweise die in US 3027398, DE 4407091, EP 258789, EP 813906, EP 1420012, WO 99/32427, WO 00/78704, WO 02/100536, WO 03/103830, WO 2005/61105, WO 2005/61106, WO 2006/136541 sowie die in der nicht vorveröffentlichten EP 09179201.0 genannten Kontakte.

Ebenfalls geeignete Kontakte sind Rhodium-haltige Kontakte. Diese enthalten Rhodium als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle sind aus der Gruppe VIIIB sind z. B. Platin, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. In diesen Katalysatoren wird vorzugsweise Rhodium alleine als Aktivmetall eingesetzt. Geeignete Rhodium-haltige Kontakte sind beispielsweise aus den zuvor für Ruthenium-haltige Katalysatoren genannten Publikationen bekannt oder können nach den dort angegebenen Vorgehensweisen hergestellt werden oder sind kommerziell erhältlich, z.B. der Katalysator Escat 34 der Fa. Engelhard.

Ebenfalls geeignete Kontakte sind Nickel-haltige Kontakte. Diese enthalten Nickel als Aktivmetall, gegebenenfalls in Kombination mit einem oder mehreren weiteren Aktivmetallen. Bevorzugte weitere Aktivmetalle sind aus den Gruppen IB, VIIB oder VIIIB des Periodensystems (CAS-Version) ausgewählt. Bei den Katalysatoren kann es sich um Vollkatalysatoren oder vorzugsweise um Trägerkatalysatoren handeln. Beispiele für weitere Aktivmetalle sind aus der Gruppe VIIIB sind z. B. Platin, Palladium, Iridium oder Cobalt oder ein Gemisch aus zwei oder mehr davon. Unter den ebenfalls verwendbaren Metallen der Nebengruppen IB und/oder VIIB des Periodensystems der Elemente sind z. B. Kupfer und/oder Rhenium geeignet. In diesen Katalysatoren wird vorzugsweise Nickel alleine als Aktivmetall eingesetzt. Geeignete Nickel-haltige Kontakte sind kommerziell erhältlich, beispielsweise BASF Katalysator Ni5249P.

Als besonders geeigneter Kontakt wird ein Trägerkatalysator eingesetzt, der als Aktivmetall Ruthenium alleine oder zusammen mit mindestens einem weiteren Aktivmetall der Nebengruppen IB, VIIB oder VIIIB des Periodensystems der Elemente (CAS-Version) auf einen Trägermaterial enthält. Bevorzugt wird Ruthenium alleine als Aktivmetall oder zusammen mit Platin oder Iridium als Aktivmetall eingesetzt; ganz besonders bevorzugt wird Ruthenium alleine als Aktivmetall eingesetzt. Als Trägermaterialien für die Ruthenium-haltigen Trägerkatalysatoren kommen grundsätzlich die vorgenannten Trägermaterialien in Betracht. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen. Bevorzugt sind Trägermaterialien mit einer spezifischen BET-Oberfläche, bestimmt durch N₂-Adsorbtion nach DIN 66131 von wenigstens 30 m²/g, insbesondere 50 bis 1000 m²/g. Die Menge an Aktivmetall beträgt üblicherweise 0,05 bis 10 Gew.-%, vorzugsweise 0,1 bis 3 Gew.-% und insbesondere 0,1 bis 1 Gew.-%, bezogen auf das Gesamtgewicht des Ruthenium-haltigen Trägerkatalysators.

Als ebenfalls geeigneter Kontakt wird ein Trägerkatalysator eingesetzt, der als Aktivmetall Rhodium alleine oder zusammen mit mindestens einem weiteren Aktivmetall der Nebengruppen IB, VIIB oder VIIIB des Periodensystems der Elemente (CAS-Version) auf einen Trägermaterial enthält. Bevorzugt wird Rhodium alleine als Aktivmetall oder zusammen mit Platin oder Iridium als Aktivmetall eingesetzt; ganz besonders bevorzugt wird Rhodium alleine als Aktivmetall eingesetzt. Als Trägermaterialien für die Rhodium-haltigen Trägerkatalysatoren kommen grundsätzlich die vorgenannten Trägermaterialien in Betracht. Bevorzugt sind Siliziumdioxid-haltige Trägermaterialien, insbesondere solche, die einen Gehalt an Siliziumdioxid von wenigstens 90 Gew.-%, bezogen auf das Gesamtgewicht des Trägermaterials aufweisen. Ebenfalls bevorzugt sind Aluminiumoxid-haltige Trägermaterialien, insbesondere solche, die einen Gehalt an Aluminiumoxid (gerechnet als Al₂O₃) von wenigstens 90 Gew.-%, bezogen auf das Gesamtgewicht des Trägermaterials aufweisen. Die Menge an Aktivmetall beträgt üblicherweise 0,05 bis 10 Gew.-%, bezogen auf das Gesamtgewicht des Rhodium-haltigen Trägerkatalysators.

Als ebenfalls geeigneter Kontakt wird ein Katalysator eingesetzt, der als Aktivmetall Nickel alleine oder zusammen mit mindestens einem weiteren Aktivmetall der Nebengruppen IB, VIIB oder VIIIB des Periodensystems der Elemente (CAS-Version), gegebenenfalls auf einen Trägermaterial enthält. Bevorzugt wird Nickel alleine als Aktivmetall eingesetzt. Als Trägermaterialien für die Nickel-haltigen Trägerkatalysatoren kommen grundsätzlich die vorgenannten Trägermaterialien in Betracht. Bevorzugt sind Siliciumdioxid, Aluminiumoxid und Magnesiumoxid-haltige Trägermaterialen, insbesondere solche, die zu wenigstens 90 Gew.-% aus derartigen Materialien bestehen. Die Menge an Aktivmetall beträgt üblicherweise 1 bis 90 Gew.-%, vorzugsweise 10 bis 80 Gew.-% und insbesondere 30 bis 70 Gew.-%, bezogen auf das Gesamtgewicht des Nickel-haltigen Trägerkatalysators. Bevorzugt sind auch solche Nickel-haltigen Kontakte, die im wesentlichen ausschließlich aus Aktiv-Metall bestehen, d.h. deren Menge an Aktivmetall mehr als 90 Gew.-%, z.B. 90 bis 100 Gew.-% beträgt.

Gemäß einer besonders geeigneten Variante der heterogen-katalytischen Hydrierung wird ein Schalenkatalysator eingesetzt, insbesondere ein Schalenkatalysator, welcher als Aktivmetall Ruthenium alleine oder zusammen mit mindestens einem weiteren Aktivmetall der Nebengruppen IB, VIIB oder VIIIB des Periodensystems der Elemente in den oben genannten Mengen aufweist. Derartige Schalenkatalysatoren sind insbesondere aus der WO 2006/136541 sowie in der nicht vorveröffentlichten EP 09179201.0 bekannt.

Ein solcher Schalenkatalysator ist ein Trägerkatalysator, der dadurch gekennzeichnet ist, dass sich die überwiegende Menge des bzw. der im Katalysator enthaltenen Aktivmetalle sich in der Nähe der Oberfläche des Katalysators befinden. Insbesondere liegen mindestens 60 Gew.-% besonders bevorzugt mindestens 80 Gew.-%, jeweils bezogen auf die Gesamtmenge des Aktivmetalls, bis zu einer Eindringtiefe von maximal 200 µm, d. h. in einer Schale mit einem Abstand von maximal 200 µm zur Oberfläche der Katalysatorpartikel, vor. Dagegen liegt im Inneren (Kern) des Katalysators keine oder nur eine sehr geringe Menge des Aktivmetalls vor. Ganz besonders geeignet ist ein Schalenkatalysator, in dem kein Aktivmetall im Inneren des Katalysators nachgewiesen werden kann, d. h. Aktivmetall liegt nur in der äußersten Schale, zum Beispiel in einer Zone bis zu einer Eindringtiefe von 100 bis 200 µm, vor. Die vorstehend genannten Daten können mittels SEM (scanning electron microscopy), EPMA (electron probe microanalysis) - EDXS (energy dispersive X-ray spectroscopy) ermittelt werden und stellen gemittelte Werte dar. Weitere Angaben bezüglich den vorstehend genannten Messverfahren und Techniken können zum Beispiel "Spectroscopy in Catalysis" von J. W. Niemantsverdriet, VCH, 1995 entnommen werden. Wegen weiterer Details bezüglich der Eindringtiefe an Aktivmetall wird auf die WO 2006/136541, insbesondere auf S. 7, Zeilen 6 bis 12 verwiesen.

Geeignete Schalenkatalysatoren weisen einen Gehalt an Aktivmetall im Bereich von 0,05 bis 1 Gew.%, insbesondere 0,1 bis 0,5 Gew.-%, besonders bevorzugt 0,25 bis 0,35 Gew.-%, jeweils bezogen auf das Gesamtgewicht des Katalysators auf.

Für die heterogen-katalytische Hydrierung sind Schalenkatalysatoren mit einem Trägermaterial auf Basis von Siliziumdioxid, im Allgemeinen amorphem Siliziumdioxid besonders geeignet. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden. Als Trägermaterialien kommen grundsätzlich amorphe Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z. B. MgO, CaO, TiO₂, ZrO₂, Fe₂O₃ und/oder Alkalimetalloxid. In einer bevorzugten Ausführungsform des Schalenkatalysators ist das Trägermaterial halogenfrei, insbesondere chlorfrei, d. h. der Gehalt an Halogen im Trägermaterial beträgt weniger als 500 Gew.-ppm, z. B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält. Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen. Geeignete amorphe Trägermaterialien auf Basis von Siliziumdioxid sind dem Fachmann geläufig und kommerziell erhältlich (siehe z. B. O. W. Flörke, "Silica" in Ullmann's Encyclopedia of Industrial Chemistry, 6th Edition on CD-ROM). Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer geeigneten Variante der heterogen-katalytischen Hydrierung weisen die Katalysatoren Kieselgele als Trägermaterialien auf. Je nach Ausgestaltung des Schalenkatalysators kann das Trägermaterial unterschiedliche Gestalt aufweisen. Sofern das Verfahren, in dem die Schalenkatalysatoren eingesetzt werden, als Suspensionsverfahren ausgestaltet ist, wird man zur Herstellung der Katalysatoren üblicherweise das Trägermaterial in Form eines feinteiligen Pulvers einsetzen. Vorzugsweise weist das Pulver Teilchengrößen im Bereich von 1 bis 200 µm insbesondere 1 bis 100 µm auf. Bei Einsatz des Schalenkatalysators in Katalysatorfestbetten verwendet man üblicherweise Formkörper aus dem Trägermaterial, die z. B. durch Extrudieren, Strangpressen oder Tablettieren erhältlich sind und die z. B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt. Ebenfalls bevorzugt sind Kugelförmige Trägermaterialien mit Kugeldurchmessern im Bereich von 1 bis 10 mm, insbesondere 2 bis 6 mm.

In besonders geeigneten Schalenkatalysatoren weist das Trägermaterial des Katalysators, bei dem es sich insbesondere um ein Trägermaterial auf Basis von Siliziumdioxid handelt, ein Porenvolumen im Bereich von 0,6 bis 1,0 ml/g, bevorzugt im Bereich von 0,65 bis 0,9 ml/g, beispielsweise 0,7 bis 0,8 ml/g, bestimmt durch Hg-Porosimetrie (DIN 66133), und eine BET-Oberfläche im Bereich 280 bis 500 m²/g, vorzugsweise im Bereich von 280 bis 400 m²/g, ganz besonders bevorzugt im Bereich von 300 bis 350 m²/g auf. Vorzugsweise weisen in derartigen Schalenkatalysatoren mindestens 90 % der vorhandenen Poren einen Durchmesser von 6 bis 12 nm, bevorzugt 7 bis 11 nm, besonders bevorzugt 8 bis 10 nm, auf. Der Porendurchmesser kann nach dem Fachmann bekannten Verfahren bestimmt werden, beispielsweise durch Hg-Porosimetrie oder N₂-Physisorption. Bevorzugt weisen mindestens 95 %, besonders bevorzugt mindestens 98 %, der vorhandenen Poren einen Porendurchmesser von 6 bis 12 nm, bevorzugt 7 bis 11 nm, besonders bevorzugt 8 bis 10 nm, auf. Bevorzugt liegen in diesen Schalenkatalysatoren keine Poren vor, die kleiner als 5 nm sind. Des Weiteren liegen in diesen Schalenkatalysatoren vorzugsweise keine Poren vor, die größer als 25 nm, insbesondere größer als 15 nm, sind. In diesem Zusammenhang bedeutet "keine Poren", dass mit üblichen Messverfahren, beispielsweise Hg-Porosimetrie oder N₂-Physisorption keine Poren mit diesen Durchmessern gefunden werden.

In geeigneten Schalenkatalysatoren beträgt die Dispersität des Aktivmetalls bevorzugt 30 bis 60 %, besonders bevorzugt 30 bis 50 %. Verfahren zur Messung der Dispersität des Aktivmetalls sind dem Fachmann an sich bekannt, beispielsweise durch Pulschemisorption, wobei die Bestimmung der Edelmetalldispersion (spezifische Metalloberfläche, Kristallitgröße) mit CO Pulsmethode durchgeführt wird (DIN 66136(1-3)).

Die heterogen-katalytische Hydrierung kann in der Flüssigphase oder in der Gasphase durchgeführt werden. Bevorzugt wird die heterogen-katalytische Hydrierung in der Flüssigphase durchgeführt.

Die heterogen-katalytische Hydrierung kann in Abwesenheit eines Lösungs- oder Verdünnungsmittels oder Gegenwart eines Lösungs- oder Verdünnungsmittels durchgeführt werden, d. h. es ist nicht erforderlich, die Hydrierung in Lösung durchzuführen. Als Lösungs- oder Verdünnungsmittel kann jedes geeignete Lösungsmittel oder Verdünnungsmittel eingesetzt werden. Als Lösungs- oder Verdünnungsmittel kommen grundsätzlich solche in Betracht, die die zu hydrierende organische Verbindung möglichst vollständig zu lösen vermögen oder sich mit dieser vollständig mischen und die unter den Hydrierungsbedingungen inert sind, d. h. nicht hydriert werden. Beispiele für geeignete Lösungsmittel sind cyclische und acyclische Ether mit vorzugsweise 4 bis 8 C-Atomen, z. B. Tetrahydrofuran, Dioxan, Methyl-tert.-butylether, Dimethoxyethan, Dimethoxypropan, Dimethyldiethylenglykol, aliphatische Alkohole mit vorzugsweise 1 bis 6 C-Atomen wie Methanol, Ethanol, n- oder Isopropanol, n-, 2-, iso- oder tert.-Butanol, Carbonsäureester aliphatischer Carbonsäuren mit vorzugsweise 3 bis 8 C-Atomen wie Essigsäuremethylester, Essigsäureethylester, Essigsäurepropylester oder Essigsäurebutylester, Propionsäuremethylester, Propionsäureethylester, Propionsäurebutylester sowie aliphatische Etheralkohole wie Methoxypropanol und cycloaliphatische Verbindungen wie Cyclohexan, Methylcyclohexan und Dimethylcyclohexan. Die Menge des eingesetzten Lösungs- oder Verdünnungsmittels ist nicht in besonderer Weise beschränkt und kann je nach Bedarf frei gewählt werden, wobei bei Verwendung eines Lösungsmittels jedoch solche Mengen bevorzugt sind, die zu einer 3 bis 70 gew.-%igen Lösung der zur Hydrierung vorgesehenen organischen Verbindung führen.

In einer Variante wird die heterogen-katalytische Hydrierung in Substanz durchgeführt.

Die eigentliche Hydrierung erfolgt üblicherweise in Analogie zu den bekannten Hydrierverfahren zur Hydrierung von organischen Verbindungen, die hydrierbare Gruppen aufweisen, bevorzugt zur Hydrierung einer carbocyclischen aromatischen Gruppe zu der entsprechenden carbocyclischen aliphatischen Gruppe, wie sie im eingangs genannten Stand der Technik beschrieben werden. Hierzu wird die organische Verbindung als flüssige Phase oder Gasphase, bevorzugt als flüssige Phase, mit dem Katalysator in Gegenwart von Wasserstoff in Kontakt gebracht. Die flüssige Phase kann man über ein Katalysator-Fließbett (Fließbett-Fahrweise) oder ein Katalysator-Festbett (Festbettfahrweise) geleitet werden.

Die Hydrierung kann sowohl kontinuierlich als auch diskontinuierlich ausgestaltet werden, wobei die kontinuierliche Verfahrensdurchführung bevorzugt ist. Vorzugsweise führt man die heterogen-katalytische Hydrierung in Rieselreaktoren oder in gefluteter Fahrweise nach der Festbettfahrweise durch, wobei die Durchführung in Rieselreaktoren besonders bevorzugt ist. Insbesondere wird hier die zu hydrierende Verbindung in Substanz, d. h. weitgehender Abwesenheit organischer Verdünnungsmittel eingesetzt (Lösungsmittelgehalt vorzugsweise < 10 %). Der Wasserstoff kann dabei sowohl im Gleichstrom mit der Lösung des zu hydrierenden Edukts als auch im Gegenstrom über den Katalysator geleitet werden. Die Hydrierung kann auch diskontinuierlich nach der Batchfahrweise durchgeführt werden. In diesem Fall wird man die Hydrierung vorzugsweise in einem organischen Lösungs- oder Verdünnungsmittel durchführen.

Bei einer diskontinuierlichen Durchführung der heterogen-katalytischen Hydrierung wird der Katalysator typischerweise in einer Menge eingesetzt, dass die Konzentration an Ruthenium in dem zur Hydrierung eingesetzten Reaktionsansatz im Bereich von 10 bis 10000 ppm, insbesondere im Bereich von 50 bis 5000 ppm, speziell im Bereich von 100 bis 1000 ppm liegt.

Die Hydrierung erfolgt typischerweise bei einem Wasserstoffdruck im Bereich von 5 bis 50 MPa, insbesondere im Bereich von 10 bis 30 MPa. Der Wasserstoff kann als solcher, oder verdünnt mit einer Inerte, beispielsweise Stickstoff oder Argon in den Reaktor eingespeist werden.

Die Hydrierung erfolgt typischerweise bei Temperaturen oberhalb 50 °C, insbesondere im Bereich von 100 bis 250 °C.

Zur Durchführung der Hydrierung geeignete Apparaturen sind dem Fachmann bekannt und richten sich in erster Linie nach der Fahrweise. Geeignete Apparaturen zur Durchführung einer Hydrierung nach der Hydrierung am Katalysatorfließbett und am Katalysatorfestbett sind z. B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM, bekannt.

Alternativ kann 2-Methyl-4-phenyl-2-pentanol oder ein Gemisch, das im wesentlichen aus 2-Methyl-4-phenyl-2-pentanol besteht, durch destillative Trennung von Gemischen, die 2-Methyl-4-phenyl-2-butanol und 2-Methyl-4-phenyl-2-pentanol enthalten gewonnen werden.

2-Methyl-4-phenyl-2-pentanol kann als Geruchsstoff verwendet werden, insbesondere als Geruchsstoff in kosmetischen Zusammensetzungen sowie in Wasch- oder Reinigungszusammensetzungen.

2-Methyl-4-phenyl-2-pentanol kann einer Hydrierung unterworfen werden. Hierbei erhält man in hoher Ausbeute 4-Cyclohexyl-2-methyl-2-pentanol.

4-Cyclohexyl-2-methyl-2-pentanol ist, ähnlich 4-Cyclohexyl-2-methyl-2-butanol, ein Geruchsstoff. Zudem kann es überraschenderweise zur Modifizierung der Geruchseigenschaften von anderen Geruchsstoffen, insbesondere von 4-Cyclohexyl-2-methyl-2-butanol verwendet werden. 4-Cyclohexyl-2-methyl-2-pentanol kann daher als Duft- bzw. Aromastoff verwendet werden, insbesondere in kosmetischen Zusammensetzungen sowie in Wasch- oder Reinigungszusammensetzungen.

Zusammensetzungen, die 4-Cyclohexyl-2-methyl-2-butanol und 4-Cyclohexyl-2-methyl-2-pentanol enthalten, zeichnen sich überraschenderweise dadurch aus, dass sie im Vergleich zum 4-Cyclohexyl-2-methyl-2-butanol eine blumigere Geruchsnote aufweisen.

In diesen Zusammensetzungen liegt das Gewichtsverhältnis von 4-Cyclohexyl-2-methyl-2-butanol zu 4-Cyclohexyl-2-methyl-2-pentanol in der Regel im Bereich von 50:1 bis 1000:1. Derartige Zusammensetzungen können auch geringe Mengen an 4-Cyclohexyl-2-methylbutan und gegebenenfalls 4-Cyclohexyl-2-methylpentan enthalten, die durch Überreduktion von 2-Methyl-4-phenyl-2-butanol bzw. 2-Methyl-4-phenyl-2-pentanol erhalten. Der Gewichtsanteil der Gesamtmenge an Cyclohexyl-2-methylbutan und gegebenenfalls 4-Cyclohexyl-2-methylpentan wird in der Regel 10 Gew.-%, insbesondere 5 Gew.-%, bezogen auf 4-Cyclohexyl-2-methyl-2-butanol, nicht überschreiten und liegt, sofern vorhanden im Bereich von 0,01 bis 10 Gew.-%, insbesondere im Bereich von 0,01 bis 5 Gew.-%, bezogen auf 4-Cyclohexyl-2-methyl-2-butanol. Es ist selbstverständlich auch möglich, Cyclohexyl-2-methylbutan und gegebenenfalls 4-Cyclohexyl-2-methylpentan abzutrennen, z.B. auf destillativem Wege, so dass die Gesamtmenge an Cyclohexyl-2-methylbutan und gegebenenfalls 4-Cyclohexyl-2-methylpentan weniger als 1 Gew.-%, insbesondere weniger als 0,5 Gew.-% oder weniger als 0,1 Gew.-%, bezogen auf 4-Cyclohexyl-2-methyl-2-butanol, beträgt. Eine spezielle Zusammensetzung sind Konzentrate, d.h. Zusammensetzungen, die im wesentlichen, d.h. zu wenigstens 95 Gew.-%, insbesondere wenigstens 98 Gew.-% und speziell wenigstens 99 Gew.-% oder wenigstens 99,5 Gew.-% aus 2-4-Cyclohexyl-2-methyl-2-butanol und geringen Mengen an 4-Cyclohexyl-2-methyl-2-pentanol besteht, z.B. Zusammensetzungen, worin das Gewichtsverhältnis von 4-Cyclohexyl-2-methyl-2-butanol zu 4-Cyclohexyl-2-methyl-2-pentanol im Bereich von 50:1 bis 1000:1 liegt.

Diese Konzentrate können Cyclohexyl-2-methylbutan und gegebenenfalls 4-Cyclohexyl-2-methylpentan in den oben genannten Mengen enthalten. Zusammensetzungen, in denen das Gewichtsverhältnis von 4-Cyclohexyl-2-methyl-2-butanol zu 4-Cyclohexyl-2-methyl-2-pentanol außerhalb der hier genannten Bereich liegt, können durch Vermischen von Cyclohexyl-2-methyl-2-butanol mit der gewünschten Menge an 4-Cyclohexyl-2-methyl-2-pentanol hergestellt werden.
Derartige Zusammensetzungen, insbesondere die vorgenannten Konzentrate, können aus den zuvor genannten Gründen als Duft- bzw. Aromastoffe verwendet werden, insbesondere in kosmetischen Zusammensetzungen sowie in Wasch- oder Reinigungszusammensetzungen.

Wie bereits zuvor gesagt, kann 4-Cyclohexyl-2-methyl-2-pentanol aus 2-Methyl-4-phenyl-2-pentanol hergestellt werden durch ein Verfahren, umfassend eine heterogen-katalytische Hydrierung von 2-Methyl-4-phenyl-2-pentanol an einem zur Kernhydrierung von Aromaten geeigneten Kontakt.
Hierbei kann man so vorgehen, dass man zunächst gezielt 2-Methyl-4-phenyl-2-pentanol herstellt und anschließend einer heterogen-katalytischen Hydrierung an einem zur Kernhydrierung von Aromaten geeigneten Kontakt wie zuvor beschriebenen unterwirft.

Beispiele für geeignete kosmetische Zusammensetzungen sind grundsätzlich alle kosmetischen Zusammensetzungen, die üblicherweise Duftstoffe enthalten. Hierzu zählen beispielsweise Eaux-de-Parfum, Eaux-de-Toilette, Eaux-de-Cologne, After-Shave Produkte wie Lotionen und Cremes, Pre-Shave Produkte, parfümierte Erfrischungstücher, Enthaarungs-Cremes and Lotionen, Bräunungscremes und -lotionen, Haarpflegemittel wie Shampoos, Haarspülungen, Haarfestiger, Haargele, Haartönungsmittel, Haarwachse, Haarsprays, Schaumfestiger, Haarmousses, Spitzenfluids, Egalisierungsmittel für Dauerwellen, Haarfärbe- und -bleichmittels oder "Hot-Oil-Treatments", weiterhin Hautreinigungsmittel wie Seifen, Waschgele, Duschgele, Körperpflegemittel wie Cremes, Öle, Lotionen und dergleichen für Haut, insbesondere Produkte zur Pflege der Hände, des Gesichts oder der Füße, Sonnenschutzmittel, Deodorantien and Antiperspirantien, Hautdesinfektionsmittel, Insekten-Repellents sowie dekorative kosmetische Produkte. Je nach Anwendungsgebiet können die kosmetischen Zusammensetzungen als wässrige oder alkoholische Flüssigkeit, Öl, (Aerosol-)Spray, (Aerosol-)Schaum, Mousse, Gel, Gelspray, Creme, Lotion, Puder, Tabs, oder Wachs formuliert werden.

Geeignete Mittel zur Reinigung und/oder Desinfektion von Oberflächen, sind beispielsweise Haushaltsreiniger, Neutralreiniger, Toilettenreiniger, Bodenreiniger, Teppichreiniger, Fensterreiniger, Polituren, Möbelpflegeprodukte, flüssige und feste Geschirrspülmittel, flüssige und feste Maschinenspülmittel, weiterhin Mittel zum Reinigen oder Behandeln von Textilien wie feste oder Flüssige Textilwaschmittel, Wäschenachbehandlungsmittel, Weichspüler, Bügelhilfsmittel, Textilerfrischer, Gewebe-Preconditioniermittel, Waschseifen, Waschtabletten und dergleichen.

Ferner kann 2-Methyl-4-phenyl-2-pentanol in anderen duftstoffhaltigen Produkten wie Luftreiniger, Lampenölen, Kerzen, Raumluftverbesserern und dergleichen eingesetzt werden.

Die Erfindung wird anhand der folgenden Beispiele näher erläutert:

### Herstellungsbeispiel 1: Herstellung des Hydrierkatalysators

Als Trägermaterial diente ein kugelförmiger SiO₂-Träger (Typ AF125 der BASF SE) mit einem Kugeldurchmesser von 3 bis 5 mm und einer Rütteldichte von 0,49 kg/l. Die BET-Oberfläche lag bei 337 m²/g, die Wasseraufnahme (WA) bei 0,83 ml/g. Zum Imprägnieren setze man eine 14,25 gew.-%ige Ruthenium(III)acetat-Lösung in Essigsäure von Umicore ein.

200 g Träger wurden in einem Rundkolben vorgelegt. 15 g der Rutheniumacetat-Lösung wurden mit destilliertem Wasser auf 150 ml verdünnt (90 % WA). Das Trägermaterial wurde im Destillationskolben eines Rotationsverdampfers vorgelegt, und das erste Viertel der Lösung wurde mit einem leichten Vakuum bei 3 bis 6 U/min auf das Trägermaterial gepumpt. Nach Beendigung der Zugabe wurde der Träger im Rotationsverdampfer weitere 10 Minuten bei 3 bis 6 U/min belassen, um den Katalysator zu homogenisieren. Dieser Tränkungs-Homogenisierungs-Schritt wurde dreimal wiederholt, bis die gesamte Lösung auf den Träger aufgebracht worden war. Das so behandelte Trägermaterial wurde im Drehrohrofen bei 140 °C bewegt getrocknet, anschließend 3 h bei 200 °C im Wasserstoffstrom reduziert (20 I/h H₂; 10 I/h N₂) und bei 25 °C passiviert (5 % Luft in N₂, 2 h). Der so erhaltene Katalysator A enthielt 0,34 Gew. -% Ruthenium, bezogen auf das Katalysatorgewicht.

### Beispiel 1 (Referenzbeispiel):

In einem 400 ml Autoklav wurden 2,08 g (20,0 mmol) Styrol und 51,2 g (853 mmol) Isopropanol vorgelegt. Der Autoklav wurde verschlossen und die Reaktionsmischung bei 350 °C (Eigendruck 230 bar) gerührt. Nach je 2 h Reaktionsdauer wurden erneut 2,08 g (20,0 mmol) Styrol zugegeben. Nach insgesamt 14 Zugaben, entsprechend einer Gesamtmenge an Styrol von 31,2 g (300 mmol), ließ man die Reaktionsmischung abkühlen, entspannte den Autoklav. Vor jeder Zugabe wurde eine Probe entnommen und mittels Gaschromatographie bezüglich ihrer Zusammensetzung analysiert. Auf diese Weise wurde die Selektivität der Bildung von 2-Methyl-4-phenyl-2-butanol bezüglich des eingesetzten Styrols bestimmt. Die Selektivitäten sind in der nachfolgenden Tabelle angegeben:

| | | | | | | | | |
|---|---|---|---|---|---|---|---|---|
| Styrol [mmol] | 40 | 80 | 120 | 160 | 200 | 240 | 280 | 300 |
| Reaktionsdauer [h] | 4 | 8 | 12 | 16 | 20 | 24 | 28 | 30 |
| Selektivität [%] | 90,0 | 81,8 | 77,3 | 70,9 | 67,9 | 63,9 | 59,6 | 57,3 |

Das Reaktionsgemisch wurde destillativ aufgearbeitet. Nach Abtrennen der Leichtsieder (vor allem Isopropanol) erhielt man ein Rohprodukt einer Reinheit > 75 %, welches weiterhin 2-Methyl-4-phenyl-2-pentanol (1,0 GC-Flächen-%) enthielt. Weitere Feindestillation ergab 25,7 g (0,151 mol) 4-Phenyl-2-methylbutan-2-ol, entsprechend einer Ausbeute von 50,3 %.

Ein bei der Feindestillation erhaltenes Gemisch aus 2-Methyl-4-phenyl-2-butanol (99,8 %) und 2-Methyl-4-phenyl-2-pentanol (0,11 GC-Flächen-%) zeichnete sich gegenüber dem Reinstoff 2-Methyl-4-phenyl-2-butanol geruchlich insbesondere durch eine zusätzliche angenehme schwach blumige Note aus.

### Beispiel 2 (Referenzbeispiel):

In einem 400 ml Autoklav wurden 118 g (1,96 mol) Isopropanol vorgelegt. Der Autoklav wurde verschlossen und auf 375 °C Innentemperatur (Eigendruck 244 bar) gebracht. Hierzu gab man rasch 2,06 g (19,8 mmol) Styrol und nach 15 min Reaktionsdauer gab man erneut 2,08 g (20,0 mmol) Styrol zu. Auf diese Weise gab man weitere vier Mal Styrol zu. Nach insgesamt 6 Zugaben, entsprechend einer Gesamtmenge an Styrol von 12,36 g (119 mmol), ließ man die Reaktionsmischung abkühlen und entspannte anschließend den Autoklav. Vor jeder Zugabe wurde eine Probe entnommen und mittels Gaschromatographie bezüglich ihrer Zusammensetzung analysiert. Auf diese Weise wurde die Selektivität der Bildung von 2-Methyl-4-phenyl-2-butanol bezüglich des eingesetzten Styrols bestimmt. Die Selektivitäten sind in der nachfolgenden Tabelle angegeben:

| | | | | | | |
|---|---|---|---|---|---|---|
| Styrol [mmol] | 19,8 | 38,6 | 59,4 | 79,2 | 99 | 118,8 |
| Reaktionsdauer [min] | 15 | 30 | 45 | 60 | 75 | 90 |
| Selektivität [%] | 72,1 | 62,5 | 65,5 | 65,1 | 65,2 | 63,6 |

### Beispiel 3: Kontinuierliche Fahrweise (Referenzbeispiel)

In einem 300 ml Autoklav legte man 78 g Isopropanol vor. Der Autoklav wurde verschlossen und die Reaktionsmischung wurde auf 350 °C Innentemperatur (Eigendruck 150 bar) erhitzt. Anschließend erfolgte druckkontrolliert die kontinuierliche Dosierung und Entnahme, so dass Druck und Temperatur stets konstant blieben. Zudosiert wurde eine Mischung aus Styrol und Isopropanol im Verhältnis von 1:10 (Gewichtsanteile) mit einer Flussgeschwindigkeit von 40 g/h. Entsprechend wurde am Reaktor ein Austrag von 40 g/h entnommen.

Der Verlauf der Reaktion wurde mittels Gaschromatographie verfolgt. Dabei stellte sich ein stationärer Zustand im Reaktor nach ca. 10 h ein. Der Umsatz an Styrol wurde zu 75 % bestimmt und es ergab sich ein Austrag an 2-Methyl-4-phenyl-2-butanol von 2 g/h.

### Beispiel 4 (Referenzbeispiel):

In einem 400 ml Autoklav wurden 51,2 g (853 mmol) Isopropanol vorgelegt. Der Autoklav wurde verschlossen und der Inhalt wurde bei 350 °C (Eigendruck 230 bar) gerührt. Über eine Dosierpumpe wurde Styrol mit einer Zugaberate von 1,04 g/h (10 mmol/h) über einen Zeitraum von insgesamt 20 h in den Autoklav gegeben. Anschließend ließ man die Reaktionsmischung abkühlen und entspannte den Autoklav. Im Zeitabstand von 4 h wurden Proben entnommen und mittels Gaschromatographie bezüglich ihrer Zusammensetzung analysiert. Auf diese Weise wurde die Selektivität der Bildung von 2-Methyl-4-phenyl-2-butanol bezüglich des eingesetzten Styrols bestimmt. Die Selektivitäten sind in der nachfolgenden Tabelle angegeben:

| | | | | | |
|---|---|---|---|---|---|
| Styrol [mmol] | 40 | 80 | 120 | 160 | 200 |
| Reaktionsdauer [h] | 4 | 8 | 12 | 16 | 20 |
| Druck [MPa] | 20,3 | 20,7 | 20,1 | 18,4 | 18,4 |
| Selektivität [%] | 81,8 | 71,3 | 65,1 | 62,2 | 58,0 |

### Beispiel 5 (Referenzbeispiel):

In einem 300 mL-Autoklaven wurden 10,2 g 2-Methyl-4-phenyl-2-butanol (62 mmol), gelöst in 150 mL Tetrahydrofuran und 1,7 g des Katalysators aus Herstellungsbeispiel 1 in einem Katalysatorkorb vorgelegt. Der Autoklav wurde dreimal mit Stickstoff gespült und danach wurden 12 Stunden bei 200 °C und 200 bar Wasserstoffdruck aufgepresst. Nach 6 und 12 Stunden wurde der Reaktionsfortschritt mittels Gaschromatographie analysiert (30 m Säulenmaterial DB1, Innendurchmesser: 0,25 mm, Filmdicke: 0,25 µm, Temperaturprogramm 50 °C - 5 min isotherm; 6 °C / min. → 290 °C - 219 min. isotherm). Die Produktanteile sind in der nachfolgenden Tabelle angegeben.

| | 4-Cyclohexyl-2-methyl-2-butanol | 2-Methyl-4-phenyl-2-butanol | 4-Cylohexyl-2-methylbutan |
|---|---|---|---|
| 6 Stunden | 88,9% | 0% | 5,9% |
| 12 Stunden | 88,2% | 0% | 6,8% |

Nach 6 h war bereits kein Ausgangsmaterial mehr zu detektieren. Der geringe Anteil von Nebenprodukten wie 4-Cyclohexyl-2-methylbutan belegt die hohe Selektivität der Hydrierung bezüglich der gewünschten Zielverbindung 4-Cyclohexyl-2-methyl-2-butanol.

### Beispiel 6 (Referenzbeispiel):

In einem 300 mL-Autoklaven wurden 70 g 2-Methyl-4-phenyl-2-butanol (0,43 mol) in 97 g Tetrahydrofuran gelöst und 2,2 g eines Katalysators gemäß Herstellungsbeispiel 1 in einem Katalysatorkorb vorgelegt. Der Autoklav wurde dreimal mit Stickstoff gespült. Danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C / min. → 290 °C - 219 min. isotherm). Die Zusammensetzung des Austrags nach 10 h ist in der folgenden Tabelle angegeben.

| | 4-Cyclohexyl-2-methyl-2-butanol | 2-Methyl-4-phenyl-2-butanol | 4-Cylohexyl-2-methyl butan |
|---|---|---|---|
| Zus. 10 h [FI.-%]¹) | 95,4% | 0,0% | 4,6% |

| | | | |
|---|---|---|---|
| 1) Zusammensetzung des Austrags nach 10 h | | | |

### Beispiel 7 (Referenzbeispiel):

In einem 300 mL-Autoklaven wurden 160 g 2-Methyl-4-phenyl-2-butanol (0,98 mol) und 6,4 g eines Katalysators gemäß Beispiel 1 der EP 1042273 (0,5% Ru / Al₂O₃) in einem Katalysatorkorb vorgelegt. Der Autoklav wurde dreimal mit Stickstoff gespült: Danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C/min. → 290 °C - 219 min. isotherm). Die Zusammensetzung des Austrags nach 10 h ist in der folgenden Tabelle angegeben.

| | 4-Cyclohexyl-2-methyl-2-butanol | 2-Methyl-4-phenyl-2-butanol | 4-Cylohexyl-2-methyl butan |
|---|---|---|---|
| Zus. 10 h [FI.-%]¹⁾ | 90,1% | 0,1% | 2,5% |

| | | | |
|---|---|---|---|
| 1) Zusammensetzung des Austrags nach 10 h | | | |

### Beispiel 8 (Referenzbeispiel):

In einem 300 mL-Autoklaven wurden 160 g 2-Methyl-4-phenyl-2-butanol (0,98 mol) und 0,64 g eines kommerziell verfügbaren Rh-Trägerkatalysators (Escat 34, Fa. Engelhard - 5% Rh / Al₂O₃) in einem Katalysatorkorb vorgelegt. Der Autoklav wurde dreimal mit Stickstoff gespült und danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C/min. → 290 °C - 219 min. isotherm). Die Zusammensetzung des Austrags nach 10 h ist in der folgenden Tabelle angegeben.

| | 4-Cyclohexyl-2-methyl-2-butanol | 2-Methyl-4-phenyl-2-butanol | 4-Cylohexyl-2-methyl butan |
|---|---|---|---|
| Zus. 10 h [FI.-%]¹) | 92,5% | 0,0% | 0,25% |

| | | | |
|---|---|---|---|
| 1) Zusammensetzung des Austrags nach 10 h | | | |

### Beispiel 9 (Referenzbeispiel):

In einem 300 mL-Autoklaven wurden 160 g 2-Methyl-4-phenyl-2-butanol (0,98 mol) und 0,04 g eines handelsüblichen Nickelkatalysators (Ni5249P, Fa BASF, ca. 65% Ni /SiO₂/MgO)vorgelegt. Der Autoklav wurde dreimal mit Stickstoff gespült und danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C / min. → 290 °C - 219 min. isotherm). Die Zusammensetzung des Austrags nach 10 h ist in der folgenden Tabelle angegeben.

| | 4-Cyclohexyl-2-methyl-2-butanol | 2-Methyl-4-phenyl-2-butanol | 4-Cylohexyl-2-methyl butan |
|---|---|---|---|
| Zus. 10 h [FI.-%]¹) | 86,9% | 4,6% | 1,02% |

| | | | |
|---|---|---|---|
| 1) Zusammensetzung des Austrags nach 10 h | | | |

### Beispiel 10 (Referenzbeispiel):

Die Umsetzung wurde in einer kontinuierlich betriebenen Laboranlage betrieben, die einen Hauptreaktor aus drei hintereinander geschalteten Rohrreaktoren mit Umlauf (Hauptreaktor (HR)) und einem als Rohrreaktor ausgestalteten Nachreaktor (NR) umfasst. Die Rohrreaktoren waren wie folgt mit dem gemäß Herstellungsbeispiel 1 hergestellten Katalysators A befüllt: HR: alle Rohre jeweils 7,7 g, NR: 3,25 g. Das erste und dritte Rohr des Hauptreaktors wurde in Rieselfahrweise, das zweite Rohr in Sumpffahrweise betrieben, wobei der Austrag des 3. Rohres teilweise mit dem Zulauf auf das erste Rohr gegeben wurde. Der Nachreaktor wurde im geraden Durchgang in Sumpffahrweise betrieben. 2-Methyl-4-phenyl-2-butanol (20-30 g/h; Kat-Belastung = 0,4-0,6 kg/(L×h)) wurde mit reinem Wasserstoff bei einer mittleren Temperatur von 120-180 °C im Hauptreaktor und 140-180 °C im Nachreaktor und einem konstanten Druck von 36 bar durch die Reaktorkaskade gepumpt. Der Umsatz an 2-Methyl-4-phenyl-2-butanol lag bei 100%, die Selektivität zu 4-Cyclohexyl-2-methyl-2-butanol bei 88-98%. Die Proben wurden mittels Gaschromatographie analysiert (30 m Säulenmaterial DB1 ID.: 0,25 mm, FD: 0,25 µm, 50 °C - 5 min isotherm, - 6 °C/min. → 290 °C - 219 min. isotherm). Die Hydrierung wurde über einen Zeitraum von 250 h betrieben, ohne eine Abnahme der Katalysatoraktivität zu beobachten.

### Beispiel 11: Mischung von 4-Cyclohexyl-2-methyl-2-butanol und 4-Cyclohexyl-2-methyl-2-pentanol (Referenzbeispiel)

In einem 300 mL-Autoklaven wurden 69 g einer Mischung, die aus 2-Methyl-4-phenyl-2-butanol (98,2 FI%) und 2-Methyl-4-phenyl-2-pentanol (0,6 FI%) besteht, in 95 g Tetrahydrofuran gelöst und 2,2 g eines Katalysators gemäß Herstellungsbeispiel 1 in einem Katalysatorkorb vorgelegt. Der Autoklav wurde dreimal mit Stickstoff gespült. Danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C / min. → 290 °C - 219 min. isotherm). Die Zusammensetzung des Austrags nach 10 h ist in der folgenden Tabelle angegeben.

| | 4-Cyclohexyl-2-methyl-2-butanol | 4-Cyclohexyl-2-methyl-2-pentanol | 2-Methyl-4-phenyl-2-pentanol | 2-Methyl-4-phenyl-2-butanol | 4-Cylohexyl-2-methylbutan |
|---|---|---|---|---|---|
| Zus. 10h [FI.-% ]¹⁾ | 97,4% | 0,5 % | 0,0 % | 0,0 % | 1,7 % |

| | | | | | |
|---|---|---|---|---|---|
| 1) Zusammensetzung des Austrags nach 10 h | | | | | |

Nach destillativer Abtrennung von 4-Cyclohexyl-2-methylbutan wurde eine Mischung aus 4-Cyclohexyl-2-methyl-2-butanol und 4-Cyclohexyl-2-methyl-2-pentanol erhalten, die sich gegenüber dem Reinstoff 4-Cyclohexyl-2-methyl-2-butanol insbesondere durch eine zusätzliche vorteilhafte leichte blumige Note unterschied.

### Beispiel 12.1: Herstellung von 2-Methyl-4-phenyl-2-pentanol (erfindungsgemäß):

Durch Umsetzung von α-Methylstyrol mit Isopropanol unter den in Beispiel 1 beschriebenen Bedingungen wurde nach mehrstufiger Destillation eine Mischung, die 2-Methyl-4-phenyl-2-pentanol (90,3 GC-Flächen-%) erhalten.

### Beispiel 12.2: Herstellung von 4-Cyclohexyl-2-methyl-2-pentanol aus 2-Methyl-4-phenyl-2-pentanol (Referenzbeispiel):

In einem 300 mL-Autoklaven wurden 27,7 g dieser Mischung in 132,3 g Tetrahydrofuran gelöst und 1,6 g eines Katalysators gemäß Herstellungsbeispiel 1 in einem Katalysatorkorb vorgelegt. Der Autoklav wurde dreimal mit Stickstoff gespült. Danach hydrierte man 10 Stunden bei 160°C und 160 bar Wasserstoffdruck. Der Austrag wurde mittels Gaschromatographie analysiert (30 m Säulenmaterial DB1 ID.: 0,25 mm, FD: 0,25 µm, 50 °C - 5 min isotherm, - 6 °C / min. 290 °C - 219 min. isotherm). Die Zusammensetzung des Austrags nach 10 h ist in der folgenden Tabelle angegeben.

| | 4-Cyclohexyl-2-methyl-2-pentanol | 2-Methyl-4-phenyl-2-pentanol |
|---|---|---|
| Zus. 10h [FI.-%]¹⁾ | 85,9 % | 1,4 % |

| | | |
|---|---|---|
| 1) Zusammensetzung des Austrags nach 10 h | | |

### 4-Cyclohexyl-2-methyl-2-pentanol mittels hochauflösender Massenspektrometrie (HRMS) und mittels ¹H-NMR identifiziert:

HRMS (GC-ToF-MS, FI):
[M-H₂O + H]⁺ 166,1726 (gemessen);
[M-H₂O + H]⁺ 166,1722 (berechnet für C₁₂H₂₂) Differenz +0,0004

¹H NMR (400 MHz, d⁶-dmso): δ = 4,05 (s, 1H), 2,48 (s, 2H), 0,95-1,77 (m, 12H), 1,05 (s, 6H), 0,92 (d, 3H) ppm.

## Patentansprüche

1. Verfahren zur Herstellung von 2-Methyl-4-phenyl-2-pentanol, umfassend die Umsetzung von α-Methylstyrol mit Isopropanol bei einer Temperatur im Bereich von 250 bis 500 °C und einem Druck im Bereich von 5 bis 50 MPa, wobei man 2-Methyl-4-phenyl-2-pentanol erhält, wobei das Molverhältnis des eingesetzten α-Methylstyrols zu dem eingesetzten Isopropanol im Bereich von 1 : 5 bis 1 : 100 liegt.

2. Verfahren nach Anspruch 1, wobei die Umsetzung in Abwesenheit eines Katalysators durchgeführt wird.

3. Verfahren nach einem der vorhergehenden Ansprüche, wobei zur Umsetzung im Wesentlichen keine von α-Methylstyrol und Isopropanol verschiedenen Einsatzstoffe eingesetzt werden.

4. Verfahren nach einem der vorhergehenden Ansprüche, wobei die Umsetzung in Schritt a) unter Bedingungen erfolgt, bei denen Isopropanol im überkritischen Zustand vorliegt.

5. Verfahren nach einem der vorhergehenden Ansprüche, wobei das Molverhältnis des eingesetzten α-Methylstyrols zu dem eingesetzten Isopropanol im Bereich von 1 : 40 bis 1 : 100 liegt.

6. Verfahren nach einem der vorhergehenden Ansprüche, wobei man wenigstens 80 % des Isopropanols vorlegt und wenigstens 80 % des in der Umsetzung eingesetzten α-Methylstyrols der Umsetzung unter Reaktionsbedingungen zuführt.

7. Verfahren nach Anspruch 6, wobei man das α-Methylstyrol so zuführt, dass das Molverhältnis des in der Reaktionszone befindlichen α-Methylstyrols zu dem in der Reaktionszone befindlichen Isopropanol während der Umsetzung weniger als 1 : 10 beträgt.

8. Verfahren nach einem der Ansprüche 1 bis 7, wobei man das in der Umsetzung erhaltene Reaktionsgemisch einer destillativen Aufreinigung unterwirft.

## Claims

1. A process for the production of 2-methyl-4-phenyl-2-pentanol, comprising the reaction of α-methylstyrene with isopropanol at a temperature in the range from 250 to 500°C and a pressure in the range from 5 to 50 MPa, giving 2-methyl-4-phenyl-2-pentanol, where the molar ratio of the α-methylstyrene used to the isopropanol used is in the range from 1:5 to 1:100.

2. The process according to claim 1, where the reaction is carried out in the absence of a catalyst.

3. The process according to any of the preceding claims, where the reaction in essence uses no starting materials other than α-methylstyrene and isopropanol.

4. The process according to any of the preceding claims, where in step a) the reaction takes place under conditions where isopropanol is in the supercritical state.

5. The process according to any of the preceding claims, where the molar ratio of α-methylstyrene used to isopropanol used is in the range from 1:40 to 1:100.

6. The process according to any of the preceding claims, where at least 80% of the isopropanol is used as initial charge and at least 80% of the α-methylstyrene used in the reaction is introduced into the reaction under reaction conditions.

7. The process according to claim 6, where the manner of introduction of the α-methylstyrene is such that during the reaction the molar ratio of the α-methylstyrene located in the reaction zone to the isopropanol located in the reaction zone is less than 1:10.

8. The process according to any of claims 1 to 7, where the reaction mixture obtained in the reaction is subjected to distillative purification.

## Revendications

1. Procédé de fabrication de 2-méthyl-4-phényl-2-pentanol, comprenant la mise en réaction d'a-méthylstyrène avec de l'isopropanol à une température dans la plage allant de 250 à 500 °C et une pression dans la plage allant de 5 à 50 MPa, du 2-méthyl-4-phényl-2-pentanol étant obtenu, le rapport molaire entre l'α-méthylstyréne utilisé et l'isopropanol utilisé se situant dans la plage allant de 1:5 à 1:100.

2. Procédé selon la revendication 1, dans lequel la réaction est réalisée en l'absence d'un catalyseur.

3. Procédé selon l'une quelconque des revendications précédentes, dans lequel essentiellement aucune matière première différente de l'α-méthylstyréne et de l'isopropanol n'est utilisée pour la réaction.

4. Procédé selon l'une quelconque des revendications précédentes, dans lequel la réaction à l'étape a) a lieu dans des conditions dans lesquelles l'isopropanol se présente à l'état supercritique.

5. Procédé selon l'une quelconque des revendications précédentes, dans lequel le rapport molaire entre l'a-méthylstyrène utilisé et l'isopropanol utilisé se situe dans la plage allant de 1:40 à 1:100.

6. Procédé selon l'une quelconque des revendications précédentes, dans lequel au moins 80 % de l'isopropanol est chargé initialement et au moins 80 % de l'a-méthylstyrène utilisé dans la réaction est introduit dans la réaction dans les conditions de réaction.

7. Procédé selon la revendication 6, dans lequel l'a-méthylstyrène est introduit de telle sorte que le rapport molaire entre l'α-méthylstyréne se trouvant dans la zone de réaction et l'isopropanol se trouvant dans la zone de réaction soit inférieur à 1:10 pendant la réaction.

8. Procédé selon l'une quelconque des revendications 1 à 7, dans lequel le mélange réactionnel obtenu dans la réaction est soumis à une purification par distillation.
